# EUROPÄISCHE PATENTSCHRIFT

(11) **EP 2 114 858 B1**
(45) Veröffentlichungstag und Bekanntmachung des Hinweises auf die Patenterteilung: **01.10.2014**
(21) Anmeldenummer: 08708200.4
(22) Anmeldetag: 25.01.2008
(51) Int. Cl.: C07C 209/48, C07C 209/52, C07C 211/36

(54) **VERFAHREN ZUR HERSTELLUNG VON 3-AMINOMETHYL-3,5,5-TRIMETHYLCYCLOHEXYLAMIN**
METHOD FOR PRODUCTION OF 3-AMINOMETHYL-3,5,5-TRIMETHYLCYCLOHEXYLAMINE
PROCÉDÉ DE PRODUCTION DE 3-AMINOMÉTHYL-3,5,5-TRIMÉTHYLCYCLOHEXYLAMINE

(30) Priorität: 07.03.2007 DE 102007011483
(43) Veröffentlichungstag der Anmeldung: 11.11.2009
(73) Patentinhaber: Evonik Degussa GmbH, 45128 Essen (DE)
(72) Erfinder: LETTMANN, Christian, 48653 Coesfeld (DE); GRUND, Gerda, 48653 Coesfeld (DE); LIPPE, Juergen, 45891 Gelsenkirchen (DE); KNOOP, Cord, 45721 Haltern (DE)
(86) Internationale Anmeldenummer: PCT/EP2008/050868
(87) Internationale Veröffentlichungsnummer: WO 2008/107226

(56) Entgegenhaltungen:
- WO-A-2005/039766
- DE-C1- 19 540 191
- US-A- 3 862 911

## Beschreibung

Die Erfindung betrifft ein verbessertes Verfahren zur Herstellung von 3-Aminomethyl-3,5,5-trimethylcyclohexylamin, nachfolgend Isophorondiamin oder abgekürzt IPD genannt, durch aminierende Hydrierung von 3-Cyano-3,5,5-trimethylcyclohexanon, nachfolgend Isophoronnitril oder abgekürzt IPN genannt, in Gegenwart eines geformten Hydrierkatalysators nach Raney.

Bevorzugt umfasst die Erfindung eine erste Stufe zur mindestens teilweisen Umsetzung von IPN mit Ammoniak zum Isophoronnitrilimin und eine zweite Stufe zur aminierenden Hydrierung des Reaktionsgemisches in Gegenwart eines Festbettkatalysators nach Raney. IPD findet Verwendung als Epoxidharzhärter, als Aminkomponente in Polyamiden sowie als Ausgangskomponente für Isophorondiisocyanat, das seinerseits wiederum Ausgangskomponente für Polyurethansysteme ist. IPD wird industriell bevorzugt aus IPN hergestellt. das in bekannter Weise durch die Anlagerung von Blausäure an Isophoron erhalten werden kann (z. B. DE 12 40 854B1 und DE 39 42 371).

Aktivierte Metallkatalysatoren sind in der chemischen Technik als Raney-Katalysatoren bekannt. Sie werden überwiegend als Pulverkatalysatoren bei einer Großzahl von Hydrierreaktionen eingesetzt. Raney-Katalysatoren werden aus einer Legierung des katalytisch aktiven Metalls und einer in Alkalien löslichen Legierungskomponente hergestellt. Als katalytisch aktive Komponente kommen hauptsächlich Nickel, Kobalt, Kupfer und Eisen zum Einsatz. Für die Herstellung von IPD aus IPN werden Kobalt- und Rutheniumkatalysatoren oftmals bevorzugt, da sie eine hohe Selektivität bezüglich der Bildung des gewünschten primären Diamins aufweisen. Als auslaugbarer Legierungsbestandteil findet überwiegend Aluminium Verwendung, aber auch Zink und Silizium sind geeignet. Die so genannte Raney-Legierung wird üblicherweise fein vermahlen, und anschließend wird die auslaugbare Komponente durch Auslaugen mit Alkalien, wie z. B. Natronlauge, ganz oder teilweise entfernt.

Pulverkatalysatoren haben den Nachteil, dass sie nur in Batch-Verfahren eingesetzt werden können. Es sind daher verschiedene Verfahren beschrieben worden, die die Herstellung von aktivierten Metall-Festbettkatalysatoren ermöglichen. Solche Festbettkatalysatoren nach Raney sind für die großtechnische Herstellung von IPD besonders geeignet, da sie eine kontinuierliche Prozessführung ermöglichen.

In der Patentschrift DE 19 540 191 wird ein zweistufiges Verfahren zur Herstellung von Isophorondiamin beschrieben. In diesem Prozess wird im ersten Reaktionsschritt Isophoronnitril durch Reaktion mit Ammoniak in Gegenwart eines Iminierungskatalysators in das entsprechende Imin überführt. Im zweiten Reaktionsschritt erfolgt die Hydrierung zum Isophorondiamin in Gegenwart eines geformten Raney-Katalysators auf Basis von Kobalt, wie er gemäß DE 43 45 265 und DE 43 35 360 erhalten werden kann. Der Nachteil des Verfahrens liegt darin, dass dem Katalysator metallisches Kobalt als Binder zugesetzt werden muss. Das zugesetzte Kobalt ist im Vergleich zu Raney-Kobalt wenig katalytisch aktiv und führt aufgrund des hohen Preises für Kobalt im Vergleich zu Katalysatoren, die ohne metallisches Kobalt als Bindemittel auskommen, zu hohen Katalysatorkosten.

In der EP 880 996 wird ein Verfahren zur Herstellung von IPD beschrieben, bei dem ein geformter Raney-Katalysator verwendet wird, der ohne Zusatz von metallischem Kobalt als Bindemittel hergestellt wird. Zur Herstellung dieser Katalysatoren wird eine als Pulver vorliegende Kobalt-Aluminium-Legierung mit einem hochmolekularen Polymer sowie gegebenenfalls Promotoren vermischt und anschließend zu Formkörpern geformt, z. B. durch Extrusion. Die Formkörper werden anschließend bei Temperaturen bis zu 850 °C. kalziniert. Die Temperaturbehandlung führt zur kontrollierten Zersetzung des Polymeren und zur Ausbildung eines Festbettkatalysators mit ausreichender mechanischer Stabilität. Anschließend erfolgt die Aktivierung durch Auslaugung des Aluminiums mittels Natronlauge. Nachteilig an diesem Verfahren ist, dass ein Großteil der eingesetzten Kobalt-Aluminium-Legierung ungenutzt bleibt, denn die Auslaugung des Aluminiums und somit Aktivierung des Katalysators erfolgt nur in der äußeren Schale des Formkörpers. Der Kern des Katalysators besteht weiterhin aus der eingesetzten Kobalt-Aluminium-Legierung und ist katalytisch inaktiv, sodass ein erheblicher Teil der relativ teuren Kobalt-Aluminium-Legierungen ungenutzt bleibt und lediglich als Träger für die aktivierte Raney-Metall-Schicht dient.

Eine optimierte Ausnutzung der eingesetzten Raney-Metall-Legierung wird erreicht, wenn Raney-Festbettkatalysatoren zum Einsatz kommen, die in Form von Hohlkörpern vorliegen, wie sie gemäß der Lehre der EP 1 068 900 erhalten werden können. Zur Herstellung der Katalysatoren wird ein Gemisch der erwünschten Legierung, eines organischen Bindemittels und wahlweise eines anorganischen Bindemittels gleichmäßig durch ein Fließbett aus Polystyrolkugeln gesprüht, wo es die Kugeln beschichtet. Die beschichteten Kugeln werden dann bei Temperaturen zwischen 450 und 1000 °C kalziniert, um das Polystyrol herauszubrennen und das Metall zusammenzusintern, um die Hohlform stabiler zu machen. Nach dem Kalzinieren wird der Katalysator durch Natronlauge aktiviert. Die Anwendung entsprechender Katalysatoren für die Herstellung von IPD ist in der EP 1 216 985 beschrieben. Der besondere Vorteil dieser Art von Katalysatoren liegt darin, dass ein großer Teil der eingesetzten Legierung nach der Aktivierung katalytisch aktiv ist, und somit die auf die eingesetzte Masse an Legierung bezogene Aktivität des Katalysators besonders hoch ist. Das Inventar an relativ teurer Legierung im Reaktor kann dadurch minimiert werden.

Nachteilig an den in der EP 1 216 985 beschriebenen Katalysatoren ist der vergleichsweise aufwendige Herstellungsprozess. Eine besonders kritische Phase des Herstellungsprozesses ist der Zeitraum zwischen dem Ausbrennen der Styroporkugeln und der Formierung einer stabilen Schale. Darüber hinaus besitzen die Katalysatoren aufgrund ihrer Hohlkörperstruktur eine geringere Bruchfestigkeit als Katalysatoren, die einen massiven Kern aufweisen. Ferner besitzen die Katalysatoren eine relativ geringe Schüttdichte von nur 0,3 bis 1,3 g/mL, was ihre Anwendung in Festbettreaktoren, die von unten nach oben mit Flüssigkeit durchströmt werden, einschränkt, denn die Katalysatorpartikel können durch das strömende Medium leicht in Bewegung versetzt werden, wodurch die Desaktivierung durch mechanischen Abrieb verstärkt wird.

Aufgabe der vorliegenden Erfindung ist es, ein Verfahren zur Herstellung von Isophorondiamin aus Isophoronnitril zu entwickeln, bei dem Hydrierkatalysatoren nach Raney eingesetzt werden, die möglichst wenig Metalllegierung enthalten, und trotzdem gleiche oder bessere IPD-Ausbeuten erzielt werden als mit den bisher bekannten Verfahren, in denen Hydrierkatalysatoren nach Raney eingesetzt werden.

Überraschend wurde nun gefunden, dass die gestellte Aufgabe durch Verwendung der im Dokument PCT/EP/2005/009656 beschriebenen Katalysatoren gelöst werden kann. Diese Beobachtung ist in sofern überraschend, als das man nicht zwangsläufig davon ausgehen kann, dass bei Einsatz der in der PCT/EP/2005/009656 beschriebenen Katalysatoren im speziellen Fall der aminierenden Hydrierung von IPN zu IPD die erforderlichen IPD-Ausbeuten erreicht werden.

In der PCT/EP/2005/009656 werden Raney-Festbettkatalysatoren beschrieben, die durch Aufbringen, insbesondere Aufsprühen einer Raney-Legierung auf einen Träger, wie z. B. Siliziumdioxid oder Aluminiumoxid, erhalten werden können. Durch das Aufbringen, insbesondere Aufsprühen der Legierung auf den Träger werden Formkörper erhalten, bei denen lediglich die äußere Schale aus der Legierung besteht, während der innere Kern der Formkörper aus dem verwendeten Trägermaterial besteht. Durch die Verwendung des Trägermaterials wird der spezifische Einsatz an relativ teurer Legierung minimiert. Die Aktivierung der Formkörper erfolgt in bekannter Weise durch Behandlung mit Säure oder Lauge. Die Vorteile der in der PCT/EP/2005/009656 beschriebenen Katalysatoren gegenüber den Hohlkörpern der EP 1 068 900 sind die weniger aufwendige Produktion und damit verringerte Produktionskosten, eine höhere mechanische Stabilität und eine größere Variabilität der Schüttdichte.

Gegenstand der Erfindung ist ein Verfahren zur Herstellung von Isophorondiamin durch aminierende Hydrierung von Isophoronnitril oder Isophoronnitrilimin enthaltenden Gemischen in Gegenwart von mindestens Ammoniak und Wasserstoff, bei dem ein geformter Raney-Typ-Hydrierkatalysator verwendet wird, der nach einem die folgenden Schritte umfassenden Produktionsverfahren hergestellt wurde:
1) Herstellung der Katalysatorvorstufe durch Aufbringen einer pulverförmigen Legierung auf ein Trägermaterial, wobei die Legierung mindestens aus einem Aktivmetall, und einer zweiten auslaugbaren Legierungskomponente, ausgewählt aus Aluminium, Silizium oder Zink, besteht,
2) Optional Trocknung und Kalzinierung der in Schritt 1) erhaltenen Formkörper,
3) Aktivierung der in Schritt 1) oder 2) erhaltenen Formkörper durch Säure und/oder Lauge,
4) Optional wird der in Schritt 3) aktivierte Katalysator weiter modifiziert, z. B. durch

Aufbringen von Metallen und/oder Metallsalzen und/oder Säuren bzw. Basen, und/oder durch Behandlung in reduzierender oder oxidierender Atmosphäre, mit der Maßgabe, dass der Katalysator kein Metallpulver als anorganischen Binder umfasst.

Die erfindungsgemäß zu verwendenden Katalysatoren sind erhältlich nach dem in der PCT/EP/2005/009656 beschriebenen Verfahren.

Die Herstellung der Katalysatorvorstufe erfolgt durch Aufbringen eines oder auch mehrerer Legierungspulver auf ein Trägermaterial. Bei den Trägern kann es sich um die unterschiedlichsten Materialien handeln, z. B. anorganische Oxide wie z. B. Alumina, Silika, Silika-Alumina, Magnesia, Zinkoxid, Titandioxid, Zirkondioxid sowie Mischungen dieser Oxide. Auch andere anorganische Materialien wie Keramiken, Formkörper aus Metallen, Glaskugeln, Aktivkohle, Siliziumcarbid, Calciumcarbonat und Bariumsulfat sind geeignet.

In einer bevorzugten Ausführungsform der Erfindung werden Legierungen auf Basis von Kobalt/Aluminium und/oder Nickel/Aluminium, besonders bevorzugt auf Basis von Kobalt/Nickel/Aluminium, und Träger auf Basis von Alumina, Silika und Alumina-Silika eingesetzt. Es ist vorteilhaft, wenn der Träger ein möglichst geringes Porenvolumen sowie eine relativ inerte Oberfläche aufweist, um am Trägermaterial ablaufende Nebenreaktion zu verhindern.

Die Aufbringung der Legierung auf den Träger erfolgt bevorzugt durch Aufsprühen einer flüssigen Suspension, mindestens enthaltend das oder die Legierungspulver sowie optional eine oder mehrere der folgenden Komponenten: Anorganische Binder (z. B. Ni, Co, Fe, Cu, andere Metallpulver oder anorganische Pulver), organische Binder (z. B. Polyvinylalkohol), Wasser, Promotoren, Porenbildner. Die Partikelgröße der pulverformigen Legierung liegt im Bereich zwischen 1 und 200 µm. Das Aufbringen der Suspension auf den Träger kann z. B. in einer Trommel oder einer Sprühkammer erfolgen, wobei bei erhöhter Temperatur gearbeitet werden kann, sodass eingebrachte Flüssigkeit, z. B. Wasser, bereits während dieses Präparationsschrittes entfernt wird.

Gegebenenfalls ist eine Vorbehandlung des Trägers notwendig, um die Haftung der aufzubringenden Legierung zu verbessern. Geeignet sind Verfahren, bei denen die Oberfläche des Trägers z. B. durch eine Säurebehandlung oder durch Ätzverfahren angerauht bzw. modifiziert werden. Gegebenfalls kann es von Vorteil sein, den Träger zur Modifizierung seiner Oberflächeneigenschaften mit einem Material vorzubelegen, das als eine Art Bindermittel zwischen Trägermaterial und Legierung wirkt. Als Binder können z. B. anorganische Oxide wie Aluminiumoxid, Titandioxid oder Metallpulver eingesetzt werden.

Optional werden die resultierenden Katalysatorvorstufen in einem zusätzlichen Verfahrensschritt weiter getrocknet und kalziniert, bevorzugt bei Temperaturen zwischen 100 und 1200°C, besonders bevorzugt zwischen 100 und 1000 °C.

Die erfindungsgemäß eingesetzten Katalysatoren können auch aus mehreren Schichten bestehen. Bevorzugt werden die Katalysatorvorstufen dann zwischen den einzelnen Beschichtungsschritten getrocknet, bevorzugt bei Temperaturen zwischen 60 und 150 °C.

Die Aktivierung der Katalysatorvorstufe erfolgt durch Auslaugen der löslichen Legierungskomponenten, bevorzugt mit einer wässrigen Mineralbase, wie z. B. Natronlauge. Anschließend wird der aktivierte Katalysator mit Wasser gewaschen.

Das Massenverhaltnis von auslaugbarer Legierungskomponente zu Aktivmetallkomponente in der Legierung liegt bevorzugt im Bereich von 20:80 bis 80:20. Die erfindungsgemäß einzusetzenden Katalysatoren enthalten neben der auslaugbaren Legierungskomponete und der Aktivmetallkomponente bevorzugt weitere Dotierelemente oder Promotoren, ausgewählt aus der Reihe der Ubergangsmetallgruppen IIIb bis VIIb sowie VIII, einschließlich der seltenen Erden Auch Hauptgruppenelemente sowie deren Verbindungen, insbesondere die der 1. und 2 Hauptgruppe. sind als Promotoren geeignet. Das Dotieren von Raney-Typ-Katalysatoren ist z B in den Dokumenten US 4,153,578, DE 21 01 856, DE 21 00 373 oder DE 20 53 799 beschrieben Bevorzugte Promotoren sind Magnesium, Chrom, Mangan, Eisen, Kobalt. Vanadium. Tantal. Titan, Cer, Wolfram, Rhenium, Platin, Palladium, Ruthenium, Nickel. Kupfer. Silber. Gold und/oder Molybdän. Ganz besonders bevorzugt sind Magnesium, Chrom und/oder Nickel. Die Promotoren können als Legierungsbestandteil zugesetzt werden und/oder zu einem beliebigen Zeitpunkt während der Präparation, z. B. erst nach dem Aktivierungsschritt. Die Promotoren können sowohl in elementarer Form als auch in Form ihrer Verbindungen zugesetzt werden. Der Anteil an Promotoren im Katalysator beträgt bis zu 20 % bezogen auf das Gesamtgewicht des Katalysators, bevorzugt zwischen 2 und 10 %.

Das Schüttgewicht der Katalysatoren kann in einem weiten Bereich zwischen 0,8 und 3 g/mL eingestellt werden und ist insbesondere von der Schüttdichte des Trägermaterials abhängig sowie von dessen Massenanteil am Katalysator, d. h. dem Verhältnis von Trägermasse zur Gesamtmasse des Katalysators.

In dem erfindungsgemäßen Verfahren zur Herstellung von Isophorondiamin werden die beschriebenen Katalysatoren für den Schritt der aminierenden Hydrierung von Isophoronnitril bzw. Isophoronnitrilimin eingesetzt. Dieser Prozess kann batchweise oder kontinuierlich durchgeführt werden.

Es ist möglich, das erfindungsgemäße Verfahren in einer Stufe oder in mehreren Stufen durchzuführen. Wird das Verfahren in einer Stufe durchgeführt, wird Isophoronnitril direkt in Anwesenheit von Ammoniak, Wasserstoff, dem geformten Hydrierkatalysator und gegebenenfalls weiteren Zusätzen und in An- oder Abwesenheit von organischen Lösungsmitteln aminierend hydriert. Der Begriff "in mehreren Stufen" bedeutet, dass zunächst in einem separaten Reaktor oder Reaktorabschnitt Isophoronnitril zunächst ganz oder teilweise in Isophoronnitrilimin umgewandelt wird, und dieses Isophoronnitrilimin als Reinsubstanz oder im Gemisch mit anderen Komponenten unter Anwesenheit von mindestens Ammoniak aminierend hydriert wird.

Eine bevorzugte Ausführungsform des erfindungsgemäßen Verfahrens zur Herstellung von IPD ist ein zweistufiger Prozess: In der ersten Stufe wird zumindest ein Teil des eingesetzten IPN bei An- oder Abwesenheit eines Iminierungskatalysators und/oder von Lösungsmitteln durch Reaktion mit Ammoniak in Isophoronnitrilimin umgewandelt. Das Verhältnis von Isophoronnitrilimin zu Isophoronnitril sollte nach der Iminierung größer 1, bevorzugt größer als 4 und ganz besonders bevorzugt größer als 9 sein. In der zweiten Stufe wird das Reaktionsprodukt der ersten Stufe, so wie es anfällt oder nach einer Weiterbehandlung und/oder Zugabe weiteren Ammoniaks, unter Anwesenheit von mindestens Ammoniak und Wasserstoff und in An- oder Abwesenheit eines organischen Lösungsmittel bei einer Temperatur von 20 bis 150 °C, bevorzugt 40 bis 130 °C, und einem Druck von 0,3 bis 50 MPa. bevorzugt 5 bis 30 MPa, an den erfindungsgemäß zu verwendenden Katalysatoren aminierend hydriert.

In einer weiteren bevorzugten Ausführungsform erfolgt die Umsetzung von IPN zu IPD in drei voneinander getrennten Reaktionsräumen. In dem ersten Reaktionsraum erfolgt die Umsetzung von IPN zu Isophoronnitrilimin mit überschüssigem Ammoniak an Iminbildungskatalysatoren bei Temperaturen zwischen 20 und 150 °C und Drücken zwischen 5 und 30 MPa. Im zweiten Reaktionsraum werden die entstanden Reaktionsprodukte mit Wasserstoff, in Gegenwart von überschüssigem Ammoniak an den erfindungsgemäß einzusetzenden Katalysatoren bei Temperaturen zwischen 20 und 130 °C und Drücken von 5 bis 30 MPa hydriert. Im dritten Reaktionsraum werden die entstanden Reaktionsprodukte an den erfindungsgemäß einzusetzenden Katalysatoren bei Temperaturen zwischen 100 und 160 °C und Drücken von 5 bis 30 MPa hydriert.

Um die Gleichgewichtseinstellung der Iminierungsreaktion zu beschleunigen ist es zweckmäßig, einen Iminierungskatalysator zu verwenden. Hierzu können die nach dem Stand der Technik bekannten Iminierungskatalysatoren verwendet werden. Geeignete Katalysatoren sind beispielsweise anorganische oder organische Ionenaustauscher (siehe EP 042 119), geträgerte Heteropolysäuren (siehe DE 44 26 472), acide Metalloxide, insbesondere Aluminiumoxid und Titandioxid (siehe EP0449089) Sulfonsäuregruppen enthaltende Organopolysiloxane (DE 19 627 265.3) und saure Zeolithe. Bei Verwendung eines Iminierungskatalysators kann die Reaktionstemperatur zwischen 10 und 150 °C, vorzugsweise zwischen 30 und 130 °C und ganz besonders bevorzugt zwischen 40 und 100 °C liegen. Der Druck liegt zwischen dem Eigendruck der Mischung und 50 MPa. Bevorzugt wird die Iminierungsreaktion bei dem Druck durchgeführt, bei dem auch die anschließende reduktive Aminierung durchgeführt wird.

Obwohl die Iminierung von Isophoronnitril mit flüssigem Ammoniak bevorzugt ohne Zugabe weiterer Lösungsmittel durchgeführt wird, kann auch in Anwesenheit zusätzlicher Lösungsmittel gearbeitet werden. Geeignet sind einwertige Alkohole mit 1 bis 4 C-Atomen, insbesondere Methanol sowie Ether, besonders THF, MTBE und Dioxan.

In der Iminierungsstufe werden Pro Mol eingesetztem IPN zwischen 1 und 500 Mol, bevorzugt 5 und 200 Mol, besonders bevorzugt zwischen 5 und 100 Mol Ammoniak eingesetzt. Typische Katalysatorbelastungen liegen im Bereich von 0,01 bis 10 kg IPN je kg Katalysator und Stunde, bevorzugt 0,5 bis 10 und besonders bevorzugt 0,5 bis 5 kg IPN je kg Katalysator und Stunde.

Bei der Iminierung in Gegenwart eines Iminierungskatalysators kann der Katalysator in Form eines Suspensionskatalysators oder Festbettkatalysators vorliegen. Vorteilhaft ist die Verwendung von Festbettkatalysatoren. In einer besonders bevorzugten Ausführungsform werden IPN und Ammoniak kontinuierlich von unten nach oben durch ein mit Iminierungskatalysator gefülltes Reaktionsrohr geleitet.

Der Iminierungskatalysator ist bevorzugt in einem eigenen Reaktor angeordnet. Es ist jedoch auch möglich, den Iminierungskatalysator zusammen mit dem zur aminiernden Hydrierung verwendeten Katalysator in demselben Reaktor anzuordnen.

Außer den zuvor genannten Bestandteilen des der Iminierungsstufe zuzuführenden Gemisches kann dieses zusätzlich höher oder niedriger als IPD siedende Fraktionen aus der destillativen Aufarbeitung des aus dem Rieselbettreaktor abgezogenen Reaktionsgemisches enthalten. Derartige Fraktionen können außer Resten an IPD auch solche Nebenprodukte enthalten, aus denen sich unter Reaktionsbedingungen erneut IPD bildet. Durch Rückführung solcher Fraktionen lässt sich die Ausbeute an IPD nennenswert steigern. Besonders vorteilhaft ist es, die oberhalb IPD siedende Fraktion, welche außer Resten an IPD auch 3,3,5-Trimethyl-6-imino-7-azabicyclo[3.2.1]octan als Hauptprodukt enthält, zurückzuführen. Ebenfalls besonders vorteilhaft ist es, nicht vollständig umgesetztes IPN, insbesondere Isophoronaminonitril enthaltende Fraktionen, zurückzuführen. Die Rückführungen können auch, falls dieses gewünscht ist, unmittelbar dem der Hydrierstufe zuzuführenden Reaktionsgemisch zugesetzt werden.

Die entscheidende Verbesserung des erfindungsgemäßen Verfahrens besteht in der Verwendung des bereits beschriebenen geformten Hydrierkatalysators nach Raney bei der aminierenden Hydrierung. In dem bevorzugten zweistufigen Prozess wird ein Isophoronnitrilimin enthaltendes Gemisch mit Hilfe des geformten Hydrierkatalysators hydriert. Das der Hydrierstufe zugeführte Gemisch kann unmittelbar jenes sein, welches bei der Iminierung von IPN mit Ammoniak in der der ersten Stufe anfällt, oder wie es nach Zugabe oder Entfernung von Komponenten, wie z. B. Ammoniak, organischen Lösungsmitteln, Basen, Co-Katalysatoren und/oder Wasser, erhalten wird. Bevorzugt wird die Hydrierung kontinuierlich in Festbettreaktoren durchgeführt, die in Riesel- oder Sumpffahrweise betrieben werden können. Geeignete Reaktortypen sind z. B. Schachtöfen, Hordenreaktoren oder Rohrbündelreaktoren. Es ist auch möglich, für die Hydrierung mehrere Festbettreaktoren hintereinander zu schalten, wobei jeder der Reaktoren wahlweise in Rieselbett- und Sumpffahrweise betrieben wird.

Es ist bevorzugt, dass die erfindungsgemäß einzusetzenden Hydrierkatalysatoren vor ihrem Einsatz in der Hydrierung zunächst mit Ammoniak konditioniert werden. Dazu werden die Katalysatoren mit Ammoniak oder mit Mischungen aus Ammoniak und einem oder mehrerer Lösungsmittel in Kontakt gebracht. Bevorzugt erfolgt die Konditionierung nach Einbau der Katalysatoren im Hydrierreaktor, sie kann aber auch vor Einbau der Katalysatoren erfolgen. Zur Konditionierung werden zwischen 0,2 und 3, bevorzugt 0,5 und 2 m⁵ Ammoniak pro m³ Katalysator und Stunde eingesetzt. Üblicherweise wird bei Temperaturen zwischen 20 und 150 °C, bevorzugt 40 bis 130 °C, gearbeitet. Besonders bevorzugt wird eine Temperaturrampe durchfahren, bei der der Katalysator beginnend bei mäßig erhöhter Temperatur, bevorzugt zwischen 20 und 50 °C, langsam bis auf die später für die Hydrierung gewünschte Reaktionstemperatur, bevorzugt 20 bis 150 °C, aufgeheizt wird. Die Konditionierung wird bevorzugt in Gegenwart von Wasserstoff durchgeführt, wobei der Partialdruck des verwendeten Wasserstoffs im Reaktor den Bereich von 0,1 bis 30 MPa, bevorzugt 5 bis 25 MPa, besonders bevorzugt 10 bis 20 MPa umfasst. Die Zeitspanne der Konditionierung ist abhängig von der verwendeten Ammoniakmenge und liegt bevorzugt zwischen 1 und 48 h, besonders bevorzugt zwischen 12 und 24 h.

Der für die Hydrierung erforderliche Wasserstoff kann dem Reaktor entweder im Überschuss, beispielsweise mit bis zu 10000 Moläquivalenten, zugeführt werden, oder nur in einer solchen Menge, dass der durch Reaktion verbrauchte Wasserstoff sowie der Teil des Wasserstoffs, der eingelöst im Produktstrom den Reaktor verlässt, nachgeführt wird. Bei kontinuierlicher Fahrweise kann der Wasserstoff im Gleich- oder Gegenstrom zugeführt werden.

In einer bevorzugten Ausführungsform erfolgt die Hydrierung an den erfindungsgemäß einzusetzenden Katalysatoren in flüssigem Ammoniak als Lösungsmittel. Pro Mol IPN werden zwischen 1 und 500 Mol, bevorzugt 5 und 200 Mol, besonders bevorzugt zwischen 5 und 100 Mol Ammoniak verwendet. Zweckmäßigenveise verwendet man mindestens die Ammoniakmenge, die bei der vorgelagerten Iminierung eingestellt wurde. Der Ammoniakanteil kann aber auch vor der Hydrierung durch Zugabe von zusätzlichem Ammoniak auf den gewünschten Wert erhöht werden.

Die Hydrierung erfolgt üblicherweise bei Temperaturen zwischen 20 und 150 °C, bevorzugt 40 und 130 °C, und Drücken von 0,3 bis 50 MPa, bevorzugt 5 bis 30 MPa.

Es ist auch möglich, die Hydrierung in Gegenwart der bereits bei der Iminierungsstufe genannten Lösungsmittel durchzuführen. Der wesentliche Vorteil bei Verwendung eines Lösungsmittels liegt darin, dass die Hydrierung bei niedrigeren Drücken zwischen 0,3 und 10 MPa durchgeführt werden kann.

Bei der Hydrierung von IPN bzw. Isophoronnitrilimin können zwei unterschiedliche Stereoisomere gebildet werden. Durch die Wahl eines Temperaturprofils im Hydrierschritt lässt sich das Isomerenverhältnis beeinflussen. Es ist z. B. möglich, ein IPN oder Isophoronnitrilimin enthaltendes Gemisch zunächst bei einer Temperatur zwischen 20 und 90 °C teilweise zu hydrieren, und anschließend die Reaktion in einem zweiten Schritt in einem Temperaturbereich zwischen 90 und 150 °C zu vervollständigen. Durch die Einhaltung relativ niedriger Reaktionstemperaturen im 1. Schritt kann die Selektivität zu Gunsten des cis-Isomeren verschoben werden. Die Einhaltung relativ niedriger Reaktionstemperaturen zu Beginn der Reaktion hat zudem den Vorteil, dass das thermisch labile Isophoronnitrilimin besonders schonend hydriert wird und dadurch z. B. die Abspaltung von Blausäure minimiert wird. Das intermediär gebildete Isophoronaminonitril ist thermisch deutlich stabiler und kann daher bei höheren Temperaturen hydriert werden, ohne dass die Abspaltung von Blausäure befürchtet werden muss.

Die Realisierung des gewünschten Temperaturprofils kann beispielsweise durch die Hintereinanderschaltung von zwei oder mehr getrennt voneinander beheizbaren Reaktoren erfolgen. Es ist aber auch möglich, ein ansteigendes Temperaturprofil in nur einem Hydrierreaktor zu realisieren. Besonders bevorzugt erfolgt die Durchführung der Hydrierreaktion in einem adiabatisch betriebenen Rieselbettreaktor, bei dem das Reaktionsgemisch dem Reaktor bei Temperaturen zwischen 20 und 90 °C zugeführt wird, und aufgrund der auftretenden und durch das Reaktionsgemisch aufgenommenen Reaktionswärme zwischen 90 und 150 °C wieder verlässt.

Das erforderliche Volumen der erfindungsgemäß einzusetzenden Katalysatoren richtet sich nach dem vom Betriebsdruck, der Temperatur, der Konzentration und der Katalysatoraktivität abhängigen LHSV-Wert, (liquid hourly space velocity) der eingehalten werden muss, um eine möglichst vollständige Hydrierung des eingesetzten IPN zu gewährleisten. Üblicherweise liegt der LHSV-Wert bei der Verwendung des bevorzugt einzusetzenden Gemisches aus IPN, Ammoniak und Wasserstoff zwischen 0,5 und 4 m³ IPN/Ammoniak-Mischung pro m³ Katalysator und Stunde, bevorzugt zwischen 1 und 3 m³/(m³ x h).

Das den Hydrierreaktor verlassende Reaktionsgemisch wird in an sich bekannter Weise aufgearbeitet. Diese Aufarbeitung umfasst üblicherweise Abtrennen des Ammoniaks, der Lösungsmittel oder Gemische aus Lösungsmittel und Ammoniak, falls Lösungsmittel vorhanden sind sowie Isolation des IPD.

Unabhängig davon, ob das erfindungsgemäße Verfahren in einer bevorzugten Ausführungsform durchgeführt wird oder nicht, können bei der Umsetzung eines Gemisches aus IPN, Ammoniak, Wasserstoff und gegebenenfalls Lösungsmittel noch eine oder mehrere Hydroxidbasen zugegeben werden. Die Zugabe von Hydroxidbasen kann die Ausbeute an IPD durch Verminderung der Nebenproduktbildung erhöhen. Die Zugabe der Hydroxidbase erfolgt bevorzugt vor dem Hydrierschritt, sie kann aber auch bereits vor der Iminierung zugesetzt werden. Geeignete Hydroxidbasen sind beispielsweise Alkalihydroxide oder Erdalkalihydroxide. Besonders bevorzugte Hydroxidbasen sind quaternäre Ammoniumhydroxide der allgemeinen Formel (R¹R²R³R⁴N)OH, wobei R¹ bis R⁴ gleich oder unterschiedlich sein können und für aliphatische, cycloaliphatische oder aromatische Reste stehen. Beispiele sind Tetramethyl-, Tetraethyl-, Tetra-n-propyl- und Tetra-n-butylammoniumhydroxid. Geeignete Konzentrationen sind 0,01 bis 100 mmol, bevorzugt 0,05 bis 20 mmol eines Tetraalkylammoniumhydroxids pro Mol IPN.

Es ist auch möglich, bei dem erfindungsgemäßen Verfahren einen oder mehrere Co-Katalysatoren zu verwenden. Geeignete Co-Katalysatoren sind Salze von Kobalt, Nickel, Lanthan, Cer oder Yttrium, bevorzugt Salze von Kobalt und Nickel.

### Beispiele

### Erfindungsgemäßer Katalysator

Es wird eine Beschichtungslösung hergestellt, indem man 776 g einer Co/Al/Cr/Ni-Legierung in 700 g Wasser, das Magensiumnitrat und Polyvinylalkohol enthält, suspendiert.

Diese Suspension wird sodann auf 1350 mL Glaskugeln mit einem mittleren Durchmesser von 1.5 bis 2 mm aufgesprüht. Dazu werden die Glaskugeln zunächst in einem nach oben gerichteten Luftstrom suspendiert und auf ca. 80 °C vorgewärmt. Anschließend wird die Suspension aufgespruht, wobei im Laufe des Aufsprühvorganges eine Temperatur von ca. 90 °C eingestellt wird, um das eingebrachte Wasser zu verdampfen.

Nach dem Beschichten der Glaskugeln mit der zuvor genannten Lösung, werden die Kugeln in aufwärts stromender Luft bei einer Temperatur von ca. 90 °C weiter getrocknet.

In einem zweiten Schritt werden dann 1350 mL der getrockneten, beschichteten Glaskugeln mit einer weiteren Legierungslösung beschichtet.

Es wird eine Beschichtungslosung hergestellt, indem man 675 g einer Co/Al/Cr/Ni-Legierung in 607 g Wasser mit einem Gehalt an 2 Gew.-% Polyinylalkohol suspendiert.

Die Suspension wird dann unter denselben Bedingungen wie oben beschrieben auf die bereits vorbeschichteten Glaskugeln aufgesprüht.

Nach dem zweiten Beschichtungsschritt werden die beschichteten Glaskugeln in einem Stickstoff/Luftstrom auf 900 °C erwärmt, um den Polyvinylalkohol herauszubrennen und die Legierungsteilchen zusammenzusintern. Die Kugeln werden dann in einer 20 Gew.-%igen Natronlauge für 1,5 h bei 90 °C aktiviert.

Die aktivierten Kugeln hatten einen Durchmesser von ca. 3,5 mm und eine Schalendicke von 800 - 900 µm.

### Vergleichskatalvsator

Die Herstellung eines Cobalt-Raney-Festbettkatalysators, der in Form einer Hohlkugel vorliegt, erfolgte gemäß den Lehren der Dokumente EP 1 068 900 und EP 1 216 985.

Es wird eine Beschichtungslösung hergestellt, indem man 800 g einer Co/Al/Cr/Ni-Legierung in 1000 mL Wasser, das Magnesiumnitrat und Polyvinylalkohol enthält, suspendiert.

Diese Suspension wird sodann auf 2000 mL Polystyrolkugeln mit einem mittleren Durchmesser von ca. 2 mm aufgesprüht, während diese in einem nach oben gerichteten Luftstrom suspendiert sind. Dazu werden die Styoporkugeln zunächst in einem nach oben gerichteten Luftstrom suspendiert und auf ca. 80 °C vorgewärmt. Anschließend wird die Suspension aufgesprüht, wobei im Laufe des Aufsprühvorganges eine Temperatur von ca. 90 °C eingestellt wird, um das eingebrachte Wasser zu verdampfen.

Nach dem Beschichten der Polystyrolkugeln mit der zuvor genannten Lösung werden die Kugeln in aufwärts strömender Luft bei Temperaturen bis zu 90 °C getrocknet.

In einem zweiten Schritt werden dann 1000 mL dieser getrockneten, beschichteten Polystyrolkugeln mit einer Legierungslösung weiter beschichtet. Die Lösung für die zweite Schicht besteht aus 800 g einer Co/Al/Cr/Ni-Legierung, die in 1000 mL einer wässrigen Lösung aus Magnesiumnitrat und Polyvinylalkohol suspendiert ist.

Die Suspension wird dann unter denselben Bedingungen wie oben beschrieben auf die bereits vorbeschichteten Styroporkugeln aufgesprüht.

Nach dem zweiten Beschichtungsschritt werden die beschichteten Polystyrolkugeln in einem Stickstoff/Luftstrom auf 900 °C erwärmt, um das Polystyrol herauszubrennen und die Legierungsteilchen zusammenzusintern. Die Hohlkugeln werden dann in einer 20 Gew.-%igcn Natronlauge für 1,5 h bei 80 °C aktiviert. Die erhaltenen aktivierten Hohlkugeln besitzen Durchmesser im Bereich um 3 mm und eine Manteldicke von ca. 700 µm.

### Aminierende Hydrierung von IPN mit erfindungsgemäßem Katalysator und Vergleichskatalysator

Die Vcrsuchsapparatur bestand aus einem Festbettreaktor, gefüllt mit 50 mL Ionentauscher gemäß EP 042 119 zur Katalysierung der Iminbildung aus IPN und Ammoniak, und einem nachgeschalteten Festbettreaktor, gefüllt mit 50 mL des Hydrierkatalysators. Zur besseren Vergleichmäßigung der Flüssigkeitsverteilung im Hydrierreaktor war die Katalysatorschüttung mit Siliziumcarbid der Korngröße < 300 µm verdünnt. Zur Konditionierung des Katalysators wurden 100 mL/h (60 g/h) Ammoniak bei 100 °C über das Festbett geleitet. Während der Konditionierung wurde ein Wasserstoffpartialdruck von ca. 100 bar eingestellt. Nach zwölf Stunden wurde die Konditionierung beendet. Im direkten Anschluss an die Konditionierung wurden 135 mL/h einer Lösung von 14,2 Gew.-% IPN in Ammoniak zugefahren. Der Iminierungsreaktor wurde von unten nach oben (Sumpffahrweise), der Hydrierreaktor von oben nach unten (Rieselfahrweise) durchströmt. Im Iminimrungsreaktor wurde eine Temperatur von 50 °C eingestellt, im Hydrierreaktor von 100 °C. Über ein Regelventil wurde durch Zufuhr von Wasserstoff der Druck im Hydrierreaktor bei 250 bar konstant gehalten. Die Zusammensetzung des Endproduktes wurde mittels Gaschromatographie ermittelt.

Ein Vergleich der Zusammensetzung der Produkte bei Verwendung des erfindungsgemäßen Katalysators und des Vergleichskatalysators ist in der Tabelle 1 enthalten.

**Tabelle 1**

| **Zusammensetzung des Endproduktes (GC%)¹⁾** | **Bei Verwendung des erfindungsgemäßen Katalysators** | **Bei Verwendung des Vergleichskatalysators** |
|---|---|---|
| Summe Isophorondiamin | 98,7 | 97,6 |
| Summe Isophoronaminonitril | 0,18 | 0,09 |
| Imin (2-Aza-4,6,6-trimethylbicyclo[3.2.1]-octan) | 0,26 | 0,6 |
| Amidin (3,3,5-Trimethyl-6-amino-7-aza-bicyclo[3.2.1]-octan) | 0,34 | 0,75 |
| Andere | 0,52 | 0,96 |

| | | |
|---|---|---|
| ¹⁾ In beiden Endprodukten sind zusätzlich ca. 9,7% Wasser enthalten, die nicht durch den GC erfasst werden. | | |

Die Versuche zeigen, dass die IPD-Ausbeute mit den erfindungsgemäß zu verwendenden Katalysatoren ca. 1 % höher liegt als bei Verwendung des Vergleichkatalysators.

## Patentansprüche

1. Verfahren zur Herstellung von Isophorondiamin durch aminierende Hydrierung von Isophoronnitril oder Isophoronnitrilimin oder diese enthaltende Gemische in Gegenwart von mindestens Ammoniak und Wasserstoff, bei dem ein geformter Raney-Typ-Hydrierkatalysator verwendet wird, der nach einem die folgenden Schritte umfassenden Produktionsverfahren hergestellt wurde:
1) Herstellung der Katalysatorvorstufe durch Aufbringen einer pulverförmige Legierung auf ein Trägermaterial, wobei die Legierung mindestens aus einem Aktivmetall, und einer zweiten auslaugbaren Legierungskomponente, ausgewählt aus Aluminium, Silizium oder Zink, besteht,
2) Optional Trocknung und Kalzinierung der in Schritt 1) erhaltenen Formkörper,
3) Aktivierung der in Schritt 1) oder 2) erhaltenen Formkörper durch Säure und/oder Lauge,
mit der Maßgabe, dass der Katalysator kein Metallpulver als anorganischen Binder umfasst.

2. Verfahren nach Anspruch 1,
**dadurch gekennzeichnet,**
**dass** als Aktivmetall Metalle der VIII. und/oder Ib. Gruppe des Periodensystems enthalten sind.

3. Verfahren nach mindestens einem der vorherigen Ansprüche,
**dadurch gekennzeichnet,**
**dass** als Aktivmetall Kobalt, Nickel, Eisen und/oder Kupfer enthalten sind.

4. Verfahren nach mindestens einem der vorherigen Ansprüche,
**dadurch gekennzeichnet,**
**dass** Legierungen auf Basis von Kobalt/Aluminium und/oder Nickel/Aluminium enthalten sind.

5. Verfahren nach mindestens einem der vorherigen Ansprüche,
**dadurch gekennzeichnet,**
**dass** die pulverförmige Legierung anorganische und/oder organische Bindemittel und/oder Promotoren und/oder Säuren und/oder Basen enthält.

6. Verfahren nach mindestens einem der vorherigen Ansprüche,
**dadurch gekennzeichnet,**
**dass** die pulverförmige Legierung Dotiermetalle enthält.

7. Verfahren nach mindestens einem der vorherigen Ansprüche,
**dadurch gekennzeichnet,**
**dass** als Promotoren und/oder Dotiermetalle Verbindungen der folgenden Gruppen des Periodensystems enthalten sind: IIa, IIIb, IVb, Vb, VIb, VIIb, VIII, Ib, IIb, IIIa, IVa und/oder Va.

8. Verfahren nach mindestens einem der vorherigen Ansprüche,
**dadurch gekennzeichnet,**
**dass** Promotoren ausgewählt aus Magnesium, Chrom, Mangan, Eisen, Kobalt, Vanadium, Tantal, Titan, Cer, Wolfram, Rhenium, Platin, Palladium, Ruthenium, Nickel, Kupfer, Silber, Gold und/oder Molybdän enthalten sind.

9. Verfahren nach mindestens einem der vorherigen Ansprüche,
**dadurch gekennzeichnet,**
**dass** als Träger Alumina, Silika, Silika-Alumina, Magnesia, Zinkoxid, Titandioxid, Zirkondioxid, Mischungen dieser Oxide, Keramiken, Formkörper aus Metallen, Glaskugeln, Aktivkohle, Siliziumcarbid, Calciumcarbonat und Bariumsulfat enthalten sind.

10. Verfahren nach mindestens einem der vorherigen Ansprüche,
**dadurch gekennzeichnet,**
**dass** die Partikelgröße der pulverförmigen Legierung im Bereich zwischen 1 und 200 µm liegt.

11. Verfahren nach mindestens einem der vorherigen Ansprüche,
**dadurch gekennzeichnet,**
**dass** der in Schritt 3) aktivierte Katalysator weiter modifiziert wird.

12. Verfahren nach mindestens einem der vorherigen Ansprüche,
**dadurch gekennzeichnet,**
**dass** die pulverförmige Legierung durch Aufsprühen auf ein Trägermaterial aufgebracht wird.

13. Verfahren nach mindestens einem der vorherigen Ansprüche,
**dadurch gekennzeichnet,**
**dass** der Katalysator vor der Hydrierung mit Ammoniak konditioniert wird.

14. Verfahren nach mindestens einem der vorherigen Ansprüche,
**dadurch gekennzeichnet,**
**dass** batchweise oder kontinuierlich ein- oder mehrstufig verfahren wird.

15. Verfahren nach mindestens einem der vorherigen Ansprüche,
**dadurch gekennzeichnet,**
**dass** 4) der in Schritt 3) aktivierte Katalysator weiter modifiziert wird, durch Aufbringen von Metallen und/oder Metallsalzen und/oder Säuren bzw. Basen, und/oder durch Behandlung in reduzierender oder oxidierender Atmosphäre.

16. Verfahren nach mindestens einem der vorherigen Ansprüche,
**dadurch gekennzeichnet,**
**dass** in der ersten Stufe zumindest ein Teil des eingesetzten IPN bei An- oder Abwesenheit mit Ammoniak in Isophoronnitrilimin umgewandelt wird,
in der zweiten Stufe das Reaktionsprodukt der ersten Stufe, so wie es anfällt oder nach einer Weiterbehandlung und/oder Zugabe weiteren Ammoniaks, unter Anwesenheit von mindestens Ammoniak und Wasserstoff und in An- oder Abwesenheit eines organischen Lösungsmittels bei einer Temperatur von 20 bis 150 °C, und einem Druck von 0,3 bis 50 MPa, an den erfindungsgemäß zu verwendenden Katalysator aminierend hydriert wird.

17. Verfahren nach mindestens einem der vorherigen Ansprüche,
wobei die Umsetzung von IPN zu IPD in drei voneinander getrennten Reaktionsräumen erfolgt:
In dem ersten Reaktionsraum erfolgt die Umsetzung von IPN zu Isophoronnitrilimin mit überschüssigem Ammoniak an Iminbildungskatalysatoren bei Temperaturen zwischen 20 und 150 °C und Drücken zwischen 5 und 30 MPa;
im zweiten Reaktionsraum werden die entstandenen Reaktionsprodukte mit Wasserstoff in Gegenwart von überschüssigem Ammoniak an den erfindungsgemäß einzusetzenden Katalysatoren bei Temperaturen zwischen 20 und 130 °C und Drücken von 5 bis 30 MPa hydriert;
im dritten Reaktionsraum werden die entstandenen Reaktionsprodukte an den erfindungsgemäß einzusetzenden Katalysatoren bei Temperaturen zwischen 100 und 160 °C und Drücken von 5 bis 30 MPa hydriert.

## Claims

1. Process for preparing isophoronediamine by aminating hydrogenation of isophoronenitrile or isophoronenitrileimine or mixtures comprising them in the presence of at least ammonia and hydrogen, in which a shaped Raney-type hydrogenation catalyst which has been prepared by a production process comprising the following steps is used:
1) preparing the catalyst precursor by applying a pulverulent alloy to a support material, the alloy consisting at least of one active metal, and a second leachable alloy component selected from aluminium, silicon and zinc,
2) optionally drying and calcining the shaped bodies obtained in step 1),
3) activating the shaped bodies obtained in step 1) or 2) by means of acid and/or alkali,
with the proviso that the catalyst does not comprise any metal powder as inorganic binder.

2. Process according to Claim 1,
**characterized in that**
the active metals present are metals of group VIII and/or Ib of the Periodic Table.

3. Process according to at least one of the preceding claims,
**characterized in that**
the active metal present is cobalt, nickel, iron and/or copper.

4. Process according to at least one of the preceding claims,
**characterized in that**
alloys based on cobalt/aluminium and/or nickel/aluminium are present.

5. Process according to at least one of the preceding claims,
**characterized in that**
the pulverulent alloy contains inorganic and/or organic binders and/or promoters and/or acids and/or bases.

6. Process according to at least one of the preceding claims,
**characterized in that**
the pulverulent alloy contains dopant metals.

7. Process according to at least one of the preceding claims,
**characterized in that**
the promoters and/or dopant metals present are compounds of the following groups of the Periodic Table: IIa, IIIb, IVb, Vb, VIb, VIIb, VIII, Ib, IIb, IIIa, IVa and/or Va.

8. Process according to at least one of the preceding claims,
**characterized in that**
promoters selected from magnesium, chromium, manganese, iron, cobalt, vanadium, tantalum, titanium, cerium, tungsten, rhenium, platinum, palladium, ruthenium, nickel, copper, silver, gold and/or molybdenum are present.

9. Process according to at least one of the preceding claims,
**characterized in that**
the supports present are alumina, silica, silica-alumina, magnesia, zinc oxide, titanium dioxide, zirconium dioxide, mixtures of these oxides, ceramics, shaped bodies composed of metals, glass spheres, activated carbon, silicon carbide, calcium carbonate and barium sulphate.

10. Process according to at least one of the preceding claims,
**characterized in that**
the particle size of the pulverulent alloy is in the range from 1 to 200 µm.

11. Process according to at least one of the preceding claims,
**characterized in that**
the catalyst activated in step 3) is modified further.

12. Process according to at least one of the preceding claims,
**characterized in that**
the pulverulent alloy is applied to a support material by spray application.

13. Process according to at least one of the preceding claims,
**characterized in that**
the catalyst is conditioned before the hydrogenation with ammonia.

14. Process according to at least one of the preceding claims,
**characterized in that**
it is a batchwise or continuous, single-stage or multistage process.

15. Process according to at least one of the preceding claims,
**characterized in that**
4) the catalyst activated in step 3) is modified further, by applying metals and/or metal salts and/or acids or bases, and/or by treatment in reducing or oxidizing atmosphere.

16. Process according to at least one of the preceding claims,
**characterized in that**,
in the first stage, at least a portion of the IPN used is converted to isophoronenitrileimine in the presence or absence of ammonia,
in the second stage, the reaction product of the first stage, as obtained or after a further treatment and/or addition of further ammonia, in the presence of at least ammonia and hydrogen and in the presence or absence of an organic solvent, is hydrogenated under aminating conditions over the catalyst to be used in accordance with the invention at a temperature of 20 to 150°C, and a pressure of 0.3 to 50 MPa.

17. Process according to at least one of the preceding claims,
wherein the conversion of IPN to IPD is effected in three separate reaction chambers:
in the first reaction chamber, IPN is converted to isophoronenitrileimine with excess ammonia over imine formation catalysts at temperatures from 20 to 150°C and pressures from 5 to 30 MPa;
in the second reaction chamber, the reaction products formed are hydrogenated with hydrogen in the presence of excess ammonia over the catalysts to be used in accordance with the invention at temperatures from 20 to 130°C and pressures of 5 to 30 MPa;
in the third reaction chamber, the reaction products formed are hydrogenated over the catalysts to be used in accordance with the invention at temperatures from 100 to 160°C and pressures of 5 to 30 MPa.

## Revendications

1. Procédé de production d'isophorone diamine par hydrogénation aminante d'isophorone nitrile ou d'isophorone nitrilimine ou de mélanges les contenant en présence au moins d'ammoniac et d'hydrogène, dans lequel on utilise un catalyseur d'hydrogénation du type Raney moulé, qui a été produit par un procédé de production comprenant les étapes suivantes:
1) production du précurseur de catalyseur par dépôt d'un alliage en poudre sur un matériau de support, l'alliage étant composé d'au moins un métal actif, et d'un deuxième composant d'alliage lixiviable, choisi parmi l'aluminium, le silicium ou le zinc,
2) en option, séchage et calcination des corps moulés obtenus à l'étape 1),
3) activation des corps moulés obtenus à l'étape 1) ou 2) par un acide et/ou une lessive,
avec la condition que le catalyseur ne comprenne aucune poudre métallique comme liant inorganique.

2. Procédé selon la revendication 1, **caractérisé en ce que** des métaux du groupe VIII et/ou Ib du système périodique sont contenus comme métal actif.

3. Procédé selon au moins une des revendications précédentes, **caractérisé en ce que** du cobalt, du nickel, du fer et/ou du cuivre sont contenus comme métal actif.

4. Procédé selon au moins une des revendications précédentes, **caractérisé en ce que** des alliages à base de cobalt/aluminium et/ou nickel/aluminium sont contenus.

5. Procédé selon au moins une des revendications précédentes, **caractérisé en ce que** l'alliage en poudre contient des liants inorganiques et/ou organiques et/ou des promoteurs et/ou des acides et/ou des bases.

6. Procédé selon au moins une des revendications précédentes, **caractérisé en ce que** l'alliage en poudre contient des métaux de dopage.

7. Procédé selon au moins une des revendications précédentes, **caractérisé en ce que** des composés des groupes suivants du système périodique: IIa, IIIb, IVb, Vb, VIb, VIIb, VIII, Ib, IIb, IIIa, IVa et/ou Va sont contenus comme promoteurs et/ou métaux de dopage.

8. Procédé selon au moins une des revendications précédentes, **caractérisé en ce que** des promoteurs choisis parmi: magnésium, chrome, manganèse, fer, cobalt, vanadium, tantale, titane, cérium, tungstène, rhénium, platine, palladium, ruthénium, nickel, cuivre, argent, or et/ou molybdène sont contenus.

9. Procédé selon au moins une des revendications précédentes, **caractérisé en ce que** de l'alumine, de la silice, de la silice-alumine, de la magnésie, de l'oxyde de zinc, du dioxyde de titane, du dioxyde de zirconium, des mélanges de ces oxydes, des céramiques, des corps moulés en métaux, des billes de verre, du charbon actif, du carbure de silicium, du carbonate de calcium et du sulfate de baryum sont contenus comme support.

10. Procédé selon au moins une des revendications précédentes, **caractérisé en ce que** la taille des particules de l'alliage en poudre se situe dans la plage entre 1 et 200 µm.

11. Procédé selon au moins une des revendications précédentes, **caractérisé en ce que** l'on modifie encore le catalyseur activé à l'étape 3).

12. Procédé selon au moins une des revendications précédentes, **caractérisé en ce que** l'on dépose l'alliage en poudre par pulvérisation sur un matériau de support.

13. Procédé selon au moins une des revendications précédentes, **caractérisé en ce que** l'on conditionne le catalyseur à l'ammoniac avant l'hydrogénation.

14. Procédé selon au moins une des revendications précédentes, **caractérisé en ce que** l'on opère par lots ou en continu en une ou plusieurs étapes.

15. Procédé selon au moins une des revendications précédentes, **caractérisé en ce que**
4) on modifie encore le catalyseur activé à l'étape 3), par le dépôt de métaux et/ou de sels métalliques et/ou d'acides ou de bases, et/ou par traitement dans une atmosphère réductrice ou oxydante.

16. Procédé selon au moins une des revendications précédentes, **caractérisé en ce que**
dans la première étape, on convertit au moins une partie de l'IPN utilisé en la présence ou en l'absence d'ammoniac en isophorone nitrilimine,
dans la deuxième étape, on soumet à une hydrogénation aminante le produit de réaction de la première étape, tel qu'il se présente ou après un traitement ultérieur et/ou une addition d'ammoniac supplémentaire, en présence au moins d'ammoniac et d'hydrogène, et en la présence ou l'absence d'un solvant organique à une température de 20 à 150°C, et une pression de 0,3 à 50 MPa, au catalyseur à utiliser selon l'invention.

17. Procédé selon au moins une des revendications précédentes,
dans lequel on effectue la conversion de IPN en IPD dans trois chambres de réaction séparées l'une de l'autre:
dans la première chambre de réaction, on effectue la conversion de IPN en isophorone nitrilimine avec un excès d'ammoniac sur des catalyseurs de formation d'imine à des températures comprises entre 20 et 150° et des pressions comprises entre 5 et 30 MPa;
dans la deuxième chambre de réaction, on effectue l'hydrogénation des produits de réaction formés avec de l'hydrogène en présence d'un excès d'ammoniac sur les catalyseurs à utiliser selon l'invention à des températures comprises entre 20 et 130°C et des pressions de 5 à 30 MPa;
dans la troisième chambre de réaction, on effectue l'hydrogénation des produits de réaction formés sur les catalyseurs à utiliser selon l'invention à des températures comprises entre 100 et 160°C et des pressions de 5 à 30 MPa.
